Europäisches Patentamt

European Patent Office

Office européen des brevets

(19)

(11) Veröffentlichungsnummer: **0 456 200 A1**

## (12) EUROPÄISCHE PATENTANMELDUNG

(21) Anmeldenummer: 91107429.2

(22) Anmeldetag: 07.05.91

(51) Int. Cl.⁵: **C12N 15/27**, C12P 21/02, C07K 13/00, C12N 1/21, A61K 37/02

(30) Priorität: 08.05.90 DE 4014750

(43) Veröffentlichungstag der Anmeldung:
13.11.91 Patentblatt 91/46

(84) Benannte Vertragsstaaten:
AT BE CH DE DK ES FR GB GR IT LI LU NL SE

(71) Anmelder: **BOEHRINGER MANNHEIM GMBH**
**Sandhofer Strasse 112-132**
**W-6800 Mannheim-Waldhof(DE)**

(72) Erfinder: **Schumacher, Günther, Dr.rer.nat.**
**Kapellenweg 20**
**W-8139 Bernried(DE)**
Erfinder: **Dony, Carola, Dr. rer.nat.**
**Waldschmidtstrasse 15**
**W-8130 Starnberg(DE)**

(74) Vertreter: **Huber, Bernhard, Dipl.-Chem. et al**
**Patentanwälte H. Weickmann, K. Fincke, F.A.**
**Weickmann, B. Huber, H. Liska, J.Prechtel,**
**Möhlstrasse 22**
**W-8000 München 80(DE)**

(54) Muteine des Granulozyten-Kolonie-stimulierenden Faktors (G-CSF).

(57) Ein Granulozyten-stimulierender Faktor (G-CSF) oder G-CSF-Variante unterscheidet sich von natürlichem G-CSF dadurch, daß eine oder mehrere Aminosäuren der Sequenz
50 51 52 53 54 55 56
Leu-Gly-His-Ser-Leu-Gly-Ile an Position 50 bis 56 des reifen G-CSF mit 174 Aminosäuren bzw. an Position 53 bis 59 des reifen G-CSF mit 177 Aminosäuren oder/und mindestens einer der 4 His-Reste an Position 43, 79, 156 oder 170 des reifen G-CSF mit 174 Aminosäuren bzw. an Position 46, 82, 159 oder 173 des reifen G-CSF mit 177 Aminosäuren mutagenisiert sind. Er eignet sich für die Immuntherapie.

EP 0 456 200 A1

EP 0 456 200 A1

Die Erfindung betrifft Muteine des Granulozyten-stimulierenden Faktors G-CSF in der Sequenz 50 51 52 53 54 55 56 Leu-Gly-His-Ser-Leu-Gly-Ile an Position 50 bis 56 des reifen G-CSF mit 174 Aminosäuren bzw. an Position 53 bis 59 des reifen G-CSF mit 177 Aminosäuren oder/und an mindestens einem der 4 His-Reste an Position 43, 79, 156 und 170 des reifen G-CSF mit 174 Aminosäuren bzw. an Position 46, 82, 159 oder 173 des reifen G-CSF mit 177 Aminosäuren.

Lymphokine sind involviert in der Reifung von Blutzellen. Sie stimulieren die Reifung von Knochenmark-Stammzellen zu ausdifferenzierten Zellen. G-CSF wird von aktivierten Monozyten, Makrophagen sowie von einer Reihe anderer Zellinien synthetisiert.

G-CSF wurde bis zur Homogenität aus Zellkulturüberständen der menschlichen Blasenkarzinom-Zellinie 5637 gereinigt (Welte et al., Proc. Natl. Acad. Sci 82 (1985), 1526). Die Sequenz der für nativen G-CSF kodierenden cDNA ist aus Sunza et al., Science 232 (1986), 61 bekannt. Es existieren, bedingt durch alternatives Splicen im zweiten Intron, zwei natürlich vorkommende Formen von G-CSF mit 204 bzw. 207 Aminosäuren, von denen die ersten 30 ein Signalpeptid darstellen (Lymphokines, IRL Press, Oxford, Washington D.C., Herausgeber D. Male und C. Rickwood). Das reife Protein hat ein Molekulargewicht von ca. 19,6 kD und besitzt 5 Cysteinreste, die inter- bzw. intramolekulare Disulfidbrücken ausbilden können. Bindungsstudien haben gezeigt, daß G-CSF an neutrophile Granulozyten bindet. Keine oder nur geringe Bindung wird bei erythroiden, lymphoiden eosinophilen Zellinien, sowie bei Makrophagen beobachtet. Der G-CSF-Rezeptor besteht aus einer einzigen Peptidkette mit einem Molekulargewicht von 150 kD (Nicola, Immunol. Today 8 (1987), 134). Die Anzahl der Rezeptoren pro Zelle nimmt im allgemeinen mit der Reifung der Zellen zu und kann einige 100 pro Zelle betragen. Man geht davon aus, daß Lymphokinrezeptoren aus einer extrazellulären Domäne, die den Liganden bindet, einer hydrophoben Transmembranregion und einer intrazellulären Domäne bestehen. Bindung von Lymphokinen an ihren Rezeptor können die Synthese von cyclischen Nukleotiden, Hydrolyse von Phosphatidylinositol 4,5-Bisphosphat, sowie die Aktivierung der Proteinkinase C und die Erhöhung des intrazellulären Calciumspiegels bewirken. Wie sich diese Vorgänge auf den Stoffwechsel der Zelle auswirken, ist von großem Interesse, aber zur Zeit nur wenig verstanden. Ein weiteres Ergebnis der Bindung eines Liganden an seinen Rezeptor kann eine Wanderung des Rezeptor-Ligand-Komplexes ins Zellinnere durch eine Rezeptor-abhängige Endozytose sein. Diese Art der Internalisierung ist offensichtlich auch bei Lymphokinen (z.B. G-CSF) gegeben, die Konsequenzen für den Stoffwechsel der Zelle sind jedoch noch nicht verstanden.

Da G-CSF in der Lage ist, die Population von neutrophile Granulozyten innerhalb kurzer Frist erheblich zu steigern, ergeben sich für G-CSF beträchtliche therapeutische Anwendungsfelder. So könnte G-CSF z.B. nach Chemotherapie bei Krebs, bei der die Zellen des Immunsystems zerstört werden, eingesetzt werden. Weiterhin könnte man G-CSF bei Knochenmark-Transplantationen, bei schweren Brandwunden, bei durch Immunschwäche bedingten opportunistischen Infektionen und bei Leukämie verwenden. Für unterschiedliche Therapieformen wäre es wünschenswert, aktivere, aber auch weniger aktive Formen des G-CSF zu entwickeln. Aufgabe der vorliegenden Erfindung war es demnach, durch gezielte Einführung von Punktmutationen G-CSF-Moleküle mit einem breiten Aktivitätsspektrum zu entwickeln. Dabei sollte diese Aktivitätsänderung durch möglichst geringe Änderungen in der Aminosäuresequenz erreicht werden.

Die erfindungsgemäße Aufgabe wird durch einen Granulozyten-stimulierenden Faktor (G-CSF) oder eine G-CSF-Variante gelöst, bei der eine oder mehrere Aminosäuren der Sequenz Leu-Gly-His-Ser-Leu-Gly-Ile an Position 50 bis 56 des reifen G-CSF mit 174 Aminosäuren bzw. an Position 53 bis 59 des reifen G-CSF mit 177 Aminosäuren oder/und mindestens einer der 4 His-Reste an Position 43, 79, 156 oder 170 des reifen G-CSF mit 174 Aminosäuren bzw. an Position 46, 82, 159 oder 173 des reifen G-CSF mit 177 Aminosäuren mutagenisiert sind.

Überraschenderweise ergibt die Einführung von neuen Aminosäuren G-CSF-Muteine, die ein breites Aktivitätsspektrum besitzen. Die Bestimmung der Aktivität kann beispielsweise nach Biochem. J. 253 (1988) 213-218; Exp. Hematol. 17 (1989) 116-119; Proc. Natl. Acad. Sci. USA 83 (1986) 5010 erfolgen.

Der Begriff G-CSF oder G-CSF-Variante gemäß vorliegender Erfindung beinhaltet alle natürlich vorkommenden Varianten von G-CSF mit oder ohne Leadersequenz, sowie davon abgeleitete, durch rekombinante DNA-Technologie modifizierte G-CSF-Proteine, insbesondere Fusionsproteine, die neben dem G-CSF-Anteil noch weitere Polypeptidsequenzen enthalten. Besonders bevorzugt ist in diesem Sinne ein G-CSF-Mutein mit einem N-terminalen Met-Rest an Position -1, das zur Expression in prokaryontischen Zellen geeignet ist. Ebenso bevorzugt ist eine rekombinante methioninfreie G-CSF-Variante, die gemäß PCT/EP91/00192 hergestellt werden kann. Der Begriff "mutagenisiert" bedeutet, daß die betreffende Aminosäure deletiert oder vorzugsweise durch eine andere Aminosäure substituiert ist.

Bevorzugt sind in diesem Sinne G-CSF-Muteine, bei denen eine der 7 Aminosäuren aus der Sequenz Leu-Gly-His-Ser-Leu-Gly-Ile durch eine andere Aminosäure substituiert ist. Es können jedoch auch mehr als

2

eine, insbesondere zwei Aminosäuren ausgetauscht werden.

Besonders bevorzugt ist ein G-CSF-Mutein, bei dem der Ser-Rest an Position 53 des reifen G-CSF mit 174 Aminosäuren bzw. an Position 56 des reifen G-CSF mit 177 Aminosäuren durch eine der 19 anderen Aminosäuren, insbesondere durch Thr ausgetauscht ist.

Weiterhin bevorzugt ist es, den Leu-Rest an Position 54 des reifen G-CSF mit 174 Aminosäuren bzw. an Position 57 des reifen G-CSF mit 177 Aminosäuren durch eine der 19 anderen Aminosäuren, insbesondere durch Thr zu substituieren. Dabei erhält man G-CSF-Muteine mit einer breiten Variation an G-CSF-Aktivität.

Weiterhin bevorzugt sind G-CSF-Muteine, bei denen einer der 4 His-Reste an Position 43, 79, 156 oder 170 des reifen G-CSF mit 174 Aminosäuren bzw. an Position 46, 82, 159 oder 173 des reifen G-CSF mit 177 Aminosäuren durch eine andere Aminosäure, insbesondere Gln ausgetauscht ist.

Weiterhin ein Gegenstand der Erfindung ist eine rekombinante DNA, die für ein erfindungsgemäßes G-CSF-Mutein kodiert. Ein Gegenstand der Erfindung ist auch ein rekombinanter Vektor, der mindestens eine Kopie einer erfindungsgemäßen rekombinanten DNA enthält. Bevorzugt ist dabei ein rekombinanter Vektor, der zur Genexpression in prokaryontischen Zellen geeignet ist. Vektoren dieser Art sind dem Fachmann bekannt.

Weiterhin ein Gegenstand der Erfindung ist eine Zelle, die mit einer erfindungsgemäßen rekombinanten DNA oder/und einem erfindungsgemäßen rekombinanten Vektor transformiert ist. Vorzugsweise ist diese Zelle eine prokaryontische Zelle, besonders bevorzugt eine E. coli-Zelle.

Ein Gegenstand der Erfindung ist auch ein Verfahren zur Herstellung einer erfindungsgemäßen rekombinanten DNA, bei dem man eine DNA-Sequenz, die für G-CSF oder eine G-CSF-Variante kodiert, stellenspezifisch mutagenisiert. Die molekularbiologisch üblichen Verfahren zur stellenspezifischen Mutagenese sind dem Fachmann bekannt. Vorzugsweise wird die Mutagenese unter Verwendung von synthetischen Oligonukleotiden als Mutagenese-Primer an einzelsträngiger DNA als Matrize durchgeführt. Übliche Verfahren sind z.B. in Amersham Nr. 1523 "Oligonucleotide-directed in vitro mutagenesis system"; Methods in Enzymology (Academic Press, Inc.) Vol. 154, Part E, 367-382 (1987); Analytical Biochemistry 179 (1989) 309-311 beschrieben.

Weiterhin ein Gegenstand der Erfindung ist ein Verfahren zur Gewinnung eines erfindungsgemäßen G-CSF-Muteins, bei dem man eine Zelle mit einer erfindungsgemäßen rekombinanten DNA oder/und einem erfindungsgemäßen rekombinanten Vektor transformiert, die transformierte Zelle in einem geeigneten Medium kultiviert und das Protein aus den Zellen oder dem Medium gewinnt. Die üblicherweise in der Molekularbiologie verwendeten Verfahren zur Gewinnung von rekombinanten Proteinen aus eukaryontischen oder prokaryontischen Zellen sind dem Fachmann bekannt und brauchen nicht im Detail erläutert werden.

Ein Gegenstand der Erfindung ist schließlich auch ein Arzneimittel auf Basis eines erfindungsgemäßen G-CSF-Muteins als Wirkstoff, gegebenenfalls zusammen mit üblichen pharmazeutischen Träger-, Füll- und Hilfsstoffen. Ein solches Arzneimittel ist insbesondere für die obengenannten therapeutischen Anwendungsfelder geeignet, jedoch auch für weitere therapeutische Verfahren, bei denen die Bildung von neutrophile Granulozyten angeregt werden soll.

Die folgenden Beispiele sollen die Erfindung verdeutlichen, jedoch nicht ihren Geltungsbereich einschränken.

**Beispiel 1**

Herstellung des Vektors mgl-G-CSF-Bg

Aus dem Vektor pKK 177-3 G-CSF-Bg (DSM 5867) wird das 554 bp lange EcoRI/BamHI-Fragment mit Shine-Dalgarno-Sequenz, ATG-Codon und kodierender Sequenz des G-CSF-Gens über eine blunt-end Ligierung in die NcoI-Schnittstelle des Vektors pPZ 07-mgl lac (WO88/09373, Figur 10) kloniert. Durch Inkubation mit Mung bean Nuklease (Pharmacia) wird zuvor das ATG-Startcodon des lac Z-Gens dieses Vektors, das nach NcoI-Verdau im überhängenden Einzelstrang liegt, verdaut. Der resultierende Vektor wird mgl-G-CSF-Bg bezeichnet.

**Beispiel 2**

Mutagenese der Aminosäure Leu (X) in der Sequenz Gly-His-Ser-Leu-Gly-Ile

Die Mutagenese wird nach den bekannten Techniken am M13-Template durchgeführt (Amersham Nr. 1523 "Oligonucleotide-directed in vitro mutagenesis system").

3

Über die Spaltstelle BstXI/AatII wird ein 251 bp langes G-CSF-cDNA-Fragment isoliert. Durch Mungbean Nuklease (Pharmacia) werden die überhängenden Einzelstränge abgedaut und das Fragment in den Vektor M13mp19 kloniert, der mit EcoRI/SmaI geschnitten wurde. Die überhängenden EcoRI-Enden wurden für eine blunt end Klonierung aufgefüllt. Nach Präparation von Einzelstrang-DNA wird das Oligonukleotid an die Einzelstrang-DNA hybridisiert und eine Verlängerung in 5'→3'-Richtung über das Oligonukleotid hinaus unter Ver-wendung von Klenow-Polymerase, Ligase und den vier Nukleotidtriphosphaten (GTP, CTP, TTP, ATP) durchgeführt. Die nun doppelsträngige DNA wird in E. coli Zellen transformiert, die ein F'-Episom tragen, so daß die Infektion durch filamentöse M13-Phagen möglich ist (z.B. JM101, erhältlich von Stratagene, LaJolla, Californien). Einzel-Plaques werden gepickt und die darin enthaltenen mutagenisierten M13-Phagen werden zur Präparation von Einzelstrang-DNA eingesetzt. Nach bekannten Techniken (z.B. Didesoxymethode nach Sanger) wird eine DNA-Sequenzierung durchgeführt und so der exakte Austausch zur gewünschten Mutation überprüft. Nach Präparation von Doppelstrang-DNA wird nun das mutierte AvaI-Fragment von G-CSF isoliert und in den Expressions-Vektor mgl-G-CSF-Bg (geschnitten mit AvaI) kloniert.

Um das vollständige G-CSF-Gen zu rekonstituieren, wird die DNA anschließend mit HindIII geschnitten, die überhängenden Enden mit Klenow-Polymerase aufgefüllt und danach mit AvaI partial verdaut, so daß die 5'AvaI Stelle im G-CSF-Gen (bei ca. 130 bp) nicht gespalten wird. Diese DNA wird mit dem etwa 240 bp großen G-CSF Fragment AvaI/BamHI (BamHI Stelle mit Klenow-Polymerase aufgefüllt) aus dem Ausgangsvektor mgl-G-CSF-Bg ligiert. Nach Transformation in E. coli JM83 erfolgt die Expression von G-CSF, auf die in der WO88/09373 beschrieben Weise.

Die verwendete cDNA weist eine Sequenz auf, die für einen G-CSF mit 175 Aminosäuren kodiert (ohne Signal-Sequenz, aber mit Met-Rest an Position -1), so daß die bevorzugte Mutation bei Leu an Position 54 der G-CSF Aminosäure-Sequenz (dabei ist der N-terminale Met-Rest nicht gezählt) gesetzt wird.

Die Sequenz der G-CSF kodierenden cDNA, die die Aminosäuren 50 bis 56 (bezogen auf den G-CSF mit 174 Aminosäuren) kodiert, lautet:

## (X)

**Leu-Gly-His-Ser-Leu-Gly-Ile**

**5'-CTC GGA CAC TCT CTG GGC ATC-3'**

Der entsprechende zu mutagenisierende komplementäre Gegenstrang lautet:
5'-GAT GCC CAG AGA GTG TCC GAG-3'
Folgende 19 dem Gegenstrang entsprechende Oligonukleotide werden zur gerichteten Mutagenese einge-setzt:

```
Wildtyp:   5'→3      GAT GCC CAG AGA GTG TCC GAG  3'


                            Met
    1.         5'      GAT GCC CAT AGA GTG TCC GAG  3'


                            Phe
    2.         5'      GAT GCC GAA AGA GTG TCC GAG  3'


                            Gln
    3.         5'      GAT GCC CTG AGA GTG TCC GAG  3'


                            Glu
    4.         5'      GAT GCC CTC AGA GTG TCC GAG  3'


                            Asp
    5.         5'      GAT GCC GTC AGA GTG TCC GAG  3'


                            Cys
    6.         5'      GAT GCC GCA AGA GTG TCC GAG  3'


                            Ala
    7.         5'      GAT GCC GGC AGA GTG TCC GAG  3'


                            Gly
    8.         5'      GAT GCC AGG AGA GTG TCC GAG  3'


                            His
    9.         5'      GAT GCC GTG AGA GTG TCC GAG  3'


                            Ile
   10.         5'      GAT GCC GAT AGA GTG TCC GAG  3'


                            Lys
   11.         5'      GAT GCC CTT AGA GTG TCC GAG  3'
```

```
                              Tyr
12.          5'      GAT GCC ATA AGA GTG TCC GAG   3'


                              Asn
13.          5'      GAT GCC GTT AGA GTG TCC GAG   3'


                              Pro
14.          5'      GAT GCC GGG AGA GTG TCC GAG   3'


                              Arg
15.          5'      GAT GCC GCG AGA GTG TCC GAG   3'


                              Ser
16.          5'      GAT GCC GGA AGA GTG TCC GAG   3'


                              Thr
17.          5'      GAT GCC GGT AGA GTG TCC GAG   3'


                              Val
18.          5'      GAT GCC GAC AGA GTG TCC GAG   3'


                              Trp
19.          5'      GAT GCC CCA AGA GTG TCC GAG   3'
```

**Beispiel 3**

Herstellung eines G-CSFs mit veränderter Aktivität

Ein im Vergleich zum Wildtyp enzymatisch aktiverer G-CSF kann durch Austausch von Serin an Position 53 in ein Threonin an Position 53 eines G-CSF mit 174 Aminosäuren (Serin in der Abfolge Gly-His-Ser-Leu-Gly) hergestellt werden. Zur Mutagenese wurde das folgende doppelsträngige Oligonukleotid verwendet:

```
                       His Thr Leu Gly Ile
5' CCC GAG GAG CTG GTG CTG CTC GGA CAC ACC CTG GGC ATC CCC TGG GCT CCC CTG AGC 3'
3'     C CTC GAC CAC GAC GAG CCT GTG TGG GAC CCG TAG GGG ACC CGA GGG GAC 5'
```

Für die Klonierung wurde das G-CSF-cDNA-Fragment (ca. 300 bp, EcoRI/EcoRV) aus dem Vektor pKK 177-3 G-CSF-Bg (DSM 5867) in die EcoRI/SmaI-Schnittstelle des Vektors pUC19 (Yannish-Perron et al. (1985), Gene 33, 103) ligiert.

Diese DNA wird mit AvaI/SacI gespalten und direkt mit dem oben beschriebenen Primerpaar nach üblichen Techniken ligiert. Aus diesem Konstrukt kann nun das mutierte BstXI/SacI-Fragment isoliert und in den Vektor pKK 177-3 G-CSF-Bg (DSM 5867) (geschnitten mit BstXI/SacI) kloniert werden. Die abschließende Erstellung des Expressionsklons erfolgt in Analogie zu Bsp. 1. Die Aktivitätsbestimmung erfolgt wie in Beispiel 5 beschrieben.

**Beispiel 4**

Veränderung der enzymatischen Eigenschaften von G-CSF durch Mutation von Aminosäuren die nicht im aktiven Zentrum liegen.

Es wird in Analogie zu bekannten Serinesterasen angenommen, daß das Serin vom aktiven Zentrum mit Histidin eine Wechselwirkung zur Ausbildung einer enzymatischen Aktivität eingeht. In der Sequenz von G-CSF sind vier Histidine vorhanden, und zwar an Position 43, 79, 156 und 170 (gezählt von der 174 A.S. Sequenz ohne Signalpeptid). Der Histidin-Rest an Position 52 (bzw. Position 55 in der 177 Aminosäuren langen Form) bleibt bei dieser Mutagenese unberücksichtigt. Dabei wird His (CCA, CTA) durch Gln (CAG) ausgetauscht. Auf dem Gegenstrang entspricht das für Gln kodierende Codon der Sequenz CTG. Wie in Beispiel 1 beschrieben, wird ein G-CSF-Fragment in M13mp19 subkloniert.

Folgende dem Gegenstrang entsprechende Oligonukleotide werden zur Mutagenese eingesetzt:

1.  5′  GCT CCT GGG CTG GCA CAG C  3′
    Histidin 43 zu Glutamin 43

2.  5′  GAA AAG GCC GCT CTG GAG TTG GCT C  3′
    Histidin 79 zu Glutamin 79

3.  5′  GCT CTG CAG CTG GCC TAG CAA CC  3′
    Histidin 156 zu Glutamin 156

4.  5′  GGG CTG CGC AAG CTG GCG TAG AAC G  3′
    Histidin 170 zu Glutamin 170

Die Analytik und Reklonierung in einen Expressionsvektor erfolgt in Analogie zu Beispiel 1.

**Beispiel 5**

Bestimmung der Aktivität von G-CSF

Die Aktivität von G-CSF wird mit der murinen Leukämie-Linie NSF60, die vollkommen G-CSF-abhängig ist, wie in Biochem. J. 253 (1988) 213-218, Exp. Hematol. 17 (1989) 116-119, Proc. Natl. Acad. Sci. USA 83 (1986) 5010 beschrieben, getestet. Damit die Faktorabhängigkeit der Zellen erhalten bleibt, enthält das Medium (RPMI Medium, Boehringer Mannheim GmbH, Best.Nr. 2099445 mit 10 % fötalem Kälberserum) der Erhaltungskultur permanent 1000 U/ml G-CSF.

Gemessen wird bei diesem Test direkt die G-CSF-stimulierende Proliferation der NSF60-Zellen durch den Einbau von $^3$H-Thymidin. Die Durchführung des Tests erfolgt folgendermaßen:

NSF60-Zellen, die sich in der exponentiellen Wachstumsphase befinden (Zelldichte maximal 1x10$^5$ Zellen/ml) werden in Mikrotiterplatten überführt (1x10$^4$ Zellen/Loch) und mit einer abnehmenden G-CSF-Konzentration kultiviert. Die maximale Dosis G-CSF in Loch 1 entspricht der Konzentration in der Erhaltungskultur (1000 U/ml, spezifische Aktivität 1x10$^8$ U/mg Protein). Die Verdünnung erfolgt in Zehnerschritten.

Nach etwa 24 Stunden Inkubation erfolgt die Zugabe von $^3$H-Thymidin (0,1 $\mu$Ci/Loch). Daraufhin werden die Zellen noch weitere 16 Stunden inkubiert.

Für die Auswertung der Tests werden die Zellen in der Mikrotiterplatte eingefroren, so daß diese lysieren. Das Zell-Lysat wird auf einen Glasfaserfilter gesaugt, gespült, getrocknet und im Scintillationszähler gemessen. Der Einbau von $^3$H-Thymidin ist proportional zur G-CSF induzierenden Proliferation der NSF60-Zellen.

**Beispiel 6**

Veränderung der Aktivität von G-CSF durch Aminosäure-Austausch im aktiven Zentrum

Ein in Aminosäureposition 54 modifizierter G-CSF kann durch Austausch von vorzugsweise einem Leucin an Position 54 in ein Threonin an Position 54 (Leu in der Abfolge Gly-His-Ser-Leu-Gly) entsprechend dem in Beispiel 3 beschriebenen Verfahren unter Verwendung eines geeigneten doppelsträngigen Oligonukleotids hergestellt werden, das an der entsprechenden Position ein für die Aminosäure Thr kodierendes Nukleinsäuretriplett (z.B. ACC) enthält. Position 54 bei der 174 Aminosäuren langen Form von G-CSF entspricht dabei der Position 57 bei der 177 Aminosäuren langen Form.

Die Aktivität einer Mutante mit 174 Aminosäuren mit Thr an Position 54 ist im NSF60 Zelltest (siehe Beispiel 5) im Vergleich zum Wildtyp G-CSF mit 174 Aminosäuren reduziert. Die Aktivität dieser G-CSF Mutante ist weiterhin im Vergleich zu einer G-CSF Mutante mit einem Aminosäure-Austausch an Position 53 von einem Serin in ein Threonin (unter Beispiel 3 beschrieben) reduziert.

**Patentansprüche**

1. Granulozyten-stimulierender Faktor (G-CSF) oder G-CSF-Variante,
   **dadurch gekennzeichnet,**
   daß eine oder mehrere Aminosäuren der Sequenz
   50 51 52 53 54 55 56
   Leu-Gly-His-Ser-Leu-Gly-Ile an Position 50 bis 56 des reifen G-CSF mit 174 Aminosäuren bzw. an Position 53 bis 59 des reifen G-CSF mit 177 Aminosäuren oder/und mindestens einer der 4 His-Reste an Position 43, 79, 156 oder 170 des reifen G-CSF mit 174 Aminosäuren bzw. an Position 46, 82, 159 oder 173 des reifen G-CSF mit 177 Aminosäuren mutagenisiert sind.

2. G-CSF Mutein nach Anspruch 1,
   **dadurch gekennzeichnet,**
   daß es an Position -1 einen N-terminalen Met-Rest enthält.

3. G-CSF Mutein nach Anspruch 1 oder 2,
   **dadurch gekennzeichnet,**
   daß eine Aminosäure der Sequenz Leu-Gly-His-Ser-Leu-Gly-Ile durch eine andere Aminosäure substituiert ist.

4. G-CSF Mutein nach einem der Ansprüche 1 bis 3,
   **dadurch gekennzeichnet,**
   daß der Ser-Rest an Position 53 des reifen G-CSF mit 174 Aminosäuren bzw. an Position 56 des reifen G-CSF mit 177 Aminosäuren durch eine andere Aminosäure substituiert ist.

5. G-CSF Mutein nach Anspruch 4,
   **dadurch gekennzeichnet,**
   daß die andere Aminosäure Thr ist.

6. G-CSF Mutein nach einem der Ansprüche 1 bis 3,
   **dadurch gekennzeichnet,**
   daß der Leu-Rest an Position 54 des reifen G-CSF mit 174 Aminosäuren bsw. an Position 57 des reifen G-CSF mit 177 Aminosäuren durch eine andere Aminosäure substituiert ist.

7. G-CSF Mutein nach Anspruch 6,
   **dadurch gekennzeichnet,**
   daß die andere Aminosäure Thr ist.

8. G-CSF Mutein nach Anspruch 1 oder 2,
   **dadurch gekennzeichnet,**
   daß einer der 4 His-Reste an Position 43, 79, 156 oder 170 des reifen G-CSF mit 174 Aminosäuren bzw. an Position 46, 82, 159 oder 173 des reifen G-CSF mit 177 Aminosäuren durch eine andere Aminosäure substituiert ist.

9. G-CSF Mutein nach Anspruch 8,
   **dadurch gekennzeichnet,**
   daß die andere Aminosäure Gln ist.

10. Rekombinante DNA,
    **dadurch gekennzeichnet,**
    daß sie für ein G-CSF Mutein nach einem der Ansprüche 1 bis 9 kodiert.

11. Rekombinanter Vektor,
    **dadurch gekennzeichnet,**
    daß er mindestens eine Kopie einer rekombinanten DNA nach Anspruch 10 enthält und zur Genexpression in prokaryontischen Zellen geeignet ist.

12. Prokaryontische Zelle,
    **dadurch gekennzeichnet,**
    daß sie mit einer rekombinanten DNA nach Anspruch 10 oder/und einem rekombinanten Vektor nach Anspruch 11 transformiert ist.

13. Verfahren zur Herstellung von rekombinanter DNA nach Anspruch 10,
    **dadurch gekennzeichnet,**
    daß man eine DNA-Sequenz, die für G-CSF oder eine G-CSF-Variante kodiert, stellenspezifisch mutagenisiert.

14. Verfahren nach Anspruch 13,
    **dadurch gekennzeichnet,**
    daß man synthetische Oligonukleotide als Mutagenese-Primer verwendet.

15. Verfahren zur Gewinnung eines Proteins mit G-CSF-Aktivität nach einem der Ansprüche 1 bis 9,
    **dadurch gekennzeichnet,**
    daß man eine Zelle mit einer rekombinanten DNA nach Anspruch 10 oder/und einem rekombinanten Vektor nach Anspruch 11 transformiert, die transformierte Zelle in einem geeigneten Medium kultiviert und das Protein aus den Zellen oder dem Medium gewinnt.

16. Arzneimittel,
    **gekennzeichnet durch**
    ein oder mehrere G-CSF Muteine nach einem der Ansprüche 1 bis 9 als Wirkstoff, gegebenenfalls zusammen mit üblichen pharmazeutischen Träger-, Füll- und Hilfsstoffen.

Europäisches
Patentamt

**EUROPÄISCHER
RECHERCHENBERICHT**

Nummer der Anmeldung

**EP 91 10 7429**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 256 843 (CETUS CORP.) <br> * Seite 8, Zeilen 4-34; Ansprüche 16-21 * <br> – – – | 1-16 | C 12 N 15/27 <br> C 12 P 21/02 <br> C 07 K 13/00 |
| A | BIOCHEMICAL AND BIOPHYSICAL RESEARCH COMM-UNICATIONS, Band 159, Nr. 1, 28. Februar 1989, Duluth, MN, US; T. KUGA et al.: "Mutagenesis of human granulocyte colony stimulating factor" <br> * Tabelle I; Seite 107, Zeile 1 * <br> – – – | 1-16 | C 12 N 1/21 <br> A 61 K 37/02 |
| A | EP-A-0 272 703 (KYOWA HAKKO KOGYO CO., LTD) <br> * Ansprüche * <br> – – – – – | 1-16 | |

| | RECHERCHIERTE SACHGEBIETE (Int. Cl.5) |
|---|---|
| | C 12 N <br> C 12 P <br> C 07 K |

Der vorliegende Recherchenbericht wurde für alle Patentansprüche erstellt

| Recherchenort | Abschlußdatum der Recherche | Prüfer |
|---|---|---|
| Den Haag | 31 Juli 91 | LE CORNEC N.D.R. |